# EUROPÄISCHE PATENTSCHRIFT

(11) **EP 1 745 772 B1**
(45) Veröffentlichungstag und Bekanntmachung des Hinweises auf die Patenterteilung: **06.01.2010**
(21) Anmeldenummer: 06117445.4
(22) Anmeldetag: 19.07.2006
(51) Int. Cl.: A61K 8/49, A61K 8/44, A61K 8/42, A61Q 17/02, A01N 47/16, A01N 37/20, A01N 37/18, A61K 8/35, A61K 8/37, A61K 8/31, A61K 8/34, A01N 37/10, A01N 37/02, A01N 43/16, A01N 35/02, A01N 27/00, A01N 31/02, A01N 43/36

(54) **Repellentien mit verstärkter Abwehrwirkung**
Insect repellents with improved activity
Insectifuges avec action fortifiée

(30) Priorität: 20.07.2005 DE 102005033845
(43) Veröffentlichungstag der Anmeldung: 24.01.2007
(60) Teilanmeldung: 09010694.9
(73) Patentinhaber: Beiersdorf AG, 20253 Hamburg (DE)
(72) Erfinder: Hamer, Gunhild, 22455, Hamburg (DE); Kröpke, Rainer, 22869, Schenefeld (DE); Schulz, Jens, 22869, Schenefeld (DE); Sibbers, Ursula, 23863, Bargfeld-Stegen (DE)

(56) Entgegenhaltungen:
- EP-A2- 1 304 924
- WO-A-03/013243
- WO-A2-03/020232
- DE-A1- 10 349 666
- DATABASE CA [Online] CHEMICAL ABSTRACTS SERVICE, COLUMBUS, OHIO, US; 2004, CHEN, BENFU ET AL: "Process for production of high-efficiency household insect repellent" XP002408572 gefunden im STN Database accession no. 2004:205571 & CN 1 381 181 CN (PEOP. REP. CHINA) 27. November 2002 (2002-11-27)
- DATABASE CA [Online] CHEMICAL ABSTRACTS SERVICE, COLUMBUS, OHIO, US; 2004, QIU, PING: "Process for preparing high-efficiency mosquito repellent" XP002408573 gefunden im STN Database accession no. 2004:21812 & CN 1 364 415 CN (PEOP. REP. CHINA) 21. August 2002 (2002-08-21)
- DATABASE WPI Week 199202 Derwent Publications Ltd., London, GB; AN 1992-013544 XP002408597 & JP 03 264504 A (OGAWA KORYO KK) 25. November 1991 (1991-11-25)
- PEPE R.C. ET AL: 'International Cosmetic Ingredient Dictionary and Handbook', 2002, WASHINGTON * Seite 2907 - Seite 2910 *
- INTERNET CITATION, [Online] Gefunden im Internet: <URL:www.inhaltsstoffe-kosmetik.de/hexyl-sa licylate-6259-76-3/> [gefunden am 2009-08-12]
- INTERNET CITATION, [Online] Gefunden im Internet: <URL:www.cosmeticsinfo.org/ingredient_detai ls.php?ingredient_id=1788> [gefunden am 2009-08-13]
- [Online] Cosmetics-CosIng alpha-ISOMETHYL IONONE
- INTERNET CITATION, [Online] Gefunden im Internet: <URL:www.basf-chemtrade.de/de/aroma-chemica ls/seite-25.html> [gefunden am 2009-08-12]
- INTERNET CITATION, [Online] Gefunden im Internet: <URL:www.inhaltsstoffe-kosmetik.de/parfums- n/> [gefunden am 2009-08-12]
- INTERNET CITATION, [Online] Gefunden im Internet: <URL:Data profile Corapan TQ from Symrise> [gefunden am 2009-08-13]
- "Sun products documentary: Benzophenone-2" COSMETICS & TOILETRIES, 1987,

## Beschreibung

Die vorliegende Erfindung betrifft eine kosmetische Zubereitung enthaltend 1-Piperidincarboxylsäure 2-(2-hydroxyethyl)-1-methylpropylester (INN: Icaridin), sowie ein oder mehrere Verbindungen gewählt aus der Gruppe Hexylsalicylat, Linalylacetat, Methylheptenon, alpha-Isomethylionon, Butylphenylmethylpropional, Coumarin, Hexylcinnamal, Diethylsuccinat, Hydroxyisohexyl 3-Cyclohexencarboxaldehyd, und Citronellylmethylcrotonat.

Insektenschutzmittel (Insektenvertreibungsmittel, Insektenabwehrmittel, Repellentien, Repellents) sind Präparate, die zur Abwehr und/oder Vertreibung von Insekten, aber auch Zecken und Milben äußerlich angewendet werden und verhindern sollen, dass diese auf der Haut aktiv werden. Insektenabwehrmittel sollen die Haut vor Belästigung durch Blut saugende oder beißende Insekten und andere Parasiten und/oder Lästlinge schützen, indem sie diese abwehren, bevor sie sich auf der Haut niederlassen. Die Mittel wirken dementsprechend nicht als Kontaktgifte, sondern nur als Abwehrmittel, da sie die Tiere nicht töten, sondern lediglich vertreiben.

Demgemäß sind im Sinne der vorliegenden Erfindung unter dem Begriff "Insektenschutzmittel" nicht nur solche Präparate zu verstehen, die gegen Insekten (insbesondere Mücken) wirksam sind. Vielmehr gilt das nachstehend Gesagte selbstverständlich auch für solche Präparate, die andere Blut saugende oder beißende Parasiten und/oder Lästlinge (z.B. Spinnen, Flöhe, Milben) abwehren oder vertreiben, auch wenn dies im Einzelfall nicht erwähnt sein mag.

Schon seit Urzeiten werden die Menschen von stechenden oder beißenden Insekten oder anderen Parasiten geplagt. Dementsprechend alt ist das Bedürfnis der Menschheit nach Insektenabwehrmitteln. Eine schon seit der Frühgeschichte bekannte Methode, lästigen oder schädlichen Insekten ihren Aufenthalt in der Nähe des Menschen unattraktiv oder unangenehm zu machen, ist das Anzünden von Feuern mit aromatisch oder streng riechenden Kräutern oder Hölzern und starker Rauchentwicklung. Auch die Behandlung der Haut mit stark riechenden Substanzen zur Abwehr von Insekten ist bereits seit der Antike bekannt. Um die letzte Jahrhundertwende war eine Reihe natürlicher etherischer Öle als Insektenabwehrmittel im Gebrauch, so beispielsweise Anisöl, Bergamottöl, Birkenholzteer, Campher, Citronellöl, Eucalyptusöl, Geraniumöl, Kiefernöle, Kokosnußöl, Lavendelöl, Muskatnußöl, Nelkenöl, Orangenblütenöl, Pfefferminzöl, Poleiöle (Pennyroyalöl), Pyrethrum, Thymianöl und Zimtöl.

Wegen ihrer trotz intensiven Geruchs unzureichenden Wirksamkeit und ihrer zum Teil mangelnden Verträglichkeit in höheren Konzentrationen wurden diese Stoffe in heutigen Insektenabwehrmitteln weitgehend durch besser wirksame synthetische Substanzen verdrängt. Es handelt sich dabei überwiegend um hoch siedende Flüssigkeiten oder niedrig schmelzende bzw. sublimierende kristalline Stoffe, die bei Raumtemperatur langsam verdampfen. Die meisten Repellent-Wirkstoffe gehören den Stoffklassen der Amide, Alkohole, Ester und Ether an.

Ein moderner Repellent-Wirkstoff ist beispielsweise der 1-Piperidincarboxylsäure 2-(2-hydroxyethyl)-1-methylpropylester (INN: Icaridin, CAS-Nummer: 119515-38-7, Elincs-Nummer: 423-210-8), der die folgende Struktur aufweist:

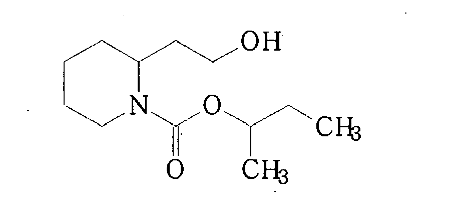

Ein weiterer häufig eingesetzter Repellent-Wirkstoff ist der 3-(N-n-Butyl-N-acetyl-amino)-propionsäureethylester (auch als Ethylbutylacetylaminopropionat oder Repellent 3535 bezeichnet), welcher sich durch die folgende Strukturformel auszeichnet:

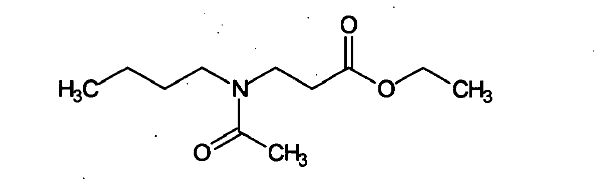

Nicht zuletzt kennt der Fachmann den Repellent-Wirkstoff N,N-Diethyl-3-methylbenzamid (Handelsbezeichnung: Meta-delphene, DEET).

Mücken, Zecken und andere Insekten im Sinne der vorliegenden Offenbarung werden in erster Linie durch eine komplexe Duftmischung aus Milchsäure, Fettsäuren und Aminosäuren angelockt, die von der menschlichen Haut verströmt werden und den Menschen wie eine Duftglocke umgeben. Es ist nun ein Nachteil an Insektenabwehrmitteln des Standes der Technik, dass derartige Zubereitungen die natürliche, menschliche Duftkomposition nicht direkt maskieren, sondern ihr vielmehr eine zusätzliche, auf Insekten abstoßend wirkende Duftnote (die des Repellent) hinzufügen. Um das anziehende und abstoßende Reizpotential auf die Insekten über einen längeren Zeitraum zugunsten der abstoßenden Reizwirkung zu verschieben, ist es nach dem Stande der Technik notwendig, relativ große Mengen an Repellent-Wirkstoffen einzusetzen.

Zubereitungen mit einer hohen Konzentration an Repellentien können jedoch bei Menschen mit besonders empfindlicher Haut in Einzelfällen zu Hautirritationen führen. Ferner haben die Repellent-Wirkstoffe einen auch für die menschliche Nase wahrnehmbaren und auf Mitmenschen nicht gerade anziehend wirkenden Eigengeruch, der die Anwendung von Insektenabwehrmitteln nicht gerade attraktiv macht. Die abweisende Wirkung der Zubereitung erstreckt sich also nicht allein auf Insekten sondern darüber hinaus unter Umständen auch auf Mitmenschen, wenn diese über einen ausgeprägten Geruchsinn verfügen.

Es waren daher die Aufgabe der vorliegenden Erfindung, die Mängel des Standes der Technik zu beseitigen und ein Insektenabwehrmittel zu entwickeln, welches mit einer reduzierten Menge an Repellent-Wirkstoffen zu einer gleichen Wirkung wie herkömmliche Insektenabwehrmittel kommt. Es war eine weitere Aufgabe der vorliegenden Erfindung, ein Insektenabwehrmittel zu entwickeln, welches ein reduziertes Hautreizungspotential aufweist. Nicht zuletzt war des die Aufgabe ein Insektenabwehrmittel zu entwickeln, welches einen für die menschliche Nase angenehm wohlriechenden Duft verströmt.

Überraschend gelöst werden die Aufgaben durch eine kosmetische Zubereitung enthaltend
a) 1-Piperidincarboxylsäure 2-(2-hydroxyethyl)-1-methylpropylester (INN: Icaridin), sowie
b) ein oder mehrere Verbindungen gewählt aus der Gruppe Hexylsalicylat, Linalylacetat, Methylheptenon, alpha-Isomethylionon, Butylphenylmethylpropipional, Coumarin, Hexylcinnamal, Diethylsuccinat, Hydroxyisohexyl 3-Cyclohexencarboxaldehyd, und Citronellylmethylcrotonat.

Dabei ist es erfindungsgemäß bevorzugt, wenn als Verbindungen aus der Gruppe b) Hexylsalicylat und/oder Linalylacetat eingesetzt werden.

Es ist erfindungsgemäß vorteilhaft, wenn die Repellentien in einer Gesamtkonzentration von 0,1 bis 40 Gewichts-% und erfindungsgemäß bevorzugt, wenn die Repellentien in einer Gesamtkonzentration von 10 bis 20 Gewichts-%, jeweils bezogen auf das Gesamtgewicht der Zubereitung, in dieser enthalten sind.

Es ist erfindungsgemäß vorteilhaft, wenn die erfindungsgemäße Zubereitung Hexylsalicylat in einer Konzentration von 1ppm bis 1,0 Gewichts-% und bevorzugt in einer Konzentration von 0,015 bis 0,065 Gewichts-%, bezogen auf das Gesamtgewicht der Zubereitung, enthält.

Es ist erfindungsgemäß vorteilhaft, wenn die erfindungsgemäße Zubereitung Linalylacetat in einer Konzentration von 1 ppm bis 1,0 Gewichts-% und bevorzugt in einer Konzentration von 0,015 bis 0,065 Gewichts-%, bezogen auf das Gesamtgewicht der Zubereitung, enthält.

Eine vorteilhafte Ausführungsform im Sinne der vorliegenden Erfindung ist eine kosmetische Zubereitung enthaltend
a) 0,1 bis 40 Gewichts-% 1-Piperidincarboxylsäure 2-(2-hydroxyethyl)-1-methylpropylester sowie
b)1 ppm bis 2,5 Gewichts-% Hexylsalicylat,
jeweils bezogen auf das Gesamtgewicht der Zubereitung.

Eine bevorzugte Ausführungsform im Sinne der vorliegenden Erfindung ist eine kosmetische Zubereitung enthaltend
a) 10 bis 20 Gewichts-% 1-Piperidincarboxylsäure 2-(2-hydroxyethyl)-1-methylpropylester sowie
b) 0,015 bis 0,065 Gewichts-% Hexylsalicylat,
jeweils bezogen auf das Gesamtgewicht der Zubereitung.

Eine vorteilhafte Ausführungsform im Sinne der vorliegenden Erfindung ist eine kosmetische Zubereitung enthaltend
a) 0,1 bis 40 Gewichts-% 1-Piperidincarboxylsäure 2-(2-hydroxyethyl)-1-methylpropylester sowie
b) 1 ppm bis 2,5 Gewichts-% Linalylacetat,
jeweils bezogen auf das Gesamtgewicht der Zubereitung.

Eine bevorzugte Ausführungsform im Sinne der vorliegenden Erfindung ist eine kosmetische Zubereitung enthaltend
a) 10 bis 20 Gewichts-% 1-Piperidincarboxylsäure 2-(2-hydroxyethyl)-1-methylpropylester sowie
b) 0,015 bis 0,0325 Gewichts-% Linalylacetat
jeweils bezogen auf das Gesamtgewicht der Zubereitung.

Die erfindungsgemäße kosmetische Zubereitung ist vorteilhaft **dadurch gekennzeichnet, das** sie in Form einer wässrigen oder wässrig-alkoholischen Lösung, einer Emulsion (W/O, O/W, W/S, S/W oder multiple Emulsion, Makro- oder Mikroemulsion), einer Dispersion, einer Pickering-Emulsion vorliegt. Dabei ist es erfindungsgemäß bevorzugt, wenn die kosmetische Zubereitung in Form einer Emulsion vorliegt.

Die Zubereitung kann erfindungsgemäß vorteilhaft in Form einer dünnflüssigen, sprühfähigen wässrigen oder wässrig-alkoholischen Lösung, in Form eines Gels, als Salbe, Creme oder Lotion (ggf. sprühbar) vorliegen.

Die Zubereitung kann erfindungsgemäß vorteilhaft auch als Spray oder als Tränkungsmedium für ein Pflaster oder Tuch eingesetzt werden. Daher sind auch Pflaster und Tücher getränkt mit der erfindungsgemäßen Zubereitung, erfindungsgemäß.

Die Wasserphase der erfindungsgemäßen Zubereitung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Eine eventuell vorhandene Ölphase kann alle herkömmlichen Bestandteile von in der Kosmetik eingesetzten Öl-, Fett- und Wachskomponenten enthalten.

Als Emulgatoren können alle aus der Kosmetik bekannten Emulgatoren und Emulgatorsysteme eingesetzt werden.

Neben der erfindungsgemäßen Wirkstoff-Kombination kann die erfindungsgemäße Zubereitung weitere kosmetische Wirk- und Pflegestoffe enthalten, beispielsweise die nach der Kosmetikverordnung zugelassenen UV-Lichtschutzfilter, und Konservierungsmittel bzw. Konservierungshelfer. Derartige Wirkstoffe können erfindungsgemäß vorteilhaft in Konzentrationen von 0,01 bis 30 Gew-%, bezogen auf das Gesamtgewicht der Zubereitung in dieser enthalten sein.

Als weitere Pflegestoffe können insbesondere Niacinamid, Panthenol, Aloe vera, Hammamelis-Extrakt, Polidocanol, Vitamin E, Vitamin E-Derivate, Vitamin A, Vitamin A-Derivate, Vitamin C, Vitamin C-Derivate, Coenzym Q10, Kreatin, Taurin, Alpha-Glycosylrutin in Konzentrationen von 0,1 bis 30 Gewichts-%, bezogen auf das Gesamtgewicht der Zubereitung eingesetzt werden.

Erfindungsgemäß besonders bevorzugt enthält die erfindungsgemäße Zubereitung als weitere Bestandteile Alpha-Hydroxysäuren und/oder deren Salze. Dabei werden erfindungsgemäß bevorzugt Milchsäure/Lactate oder Zitronensäure/Citrate in einer Konzentration von 0,01 bis 5 Gewichts-%, bezogen auf das Gesamtgewicht der Zubereitung eingesetzt.

Erfindungsgemäß ist ein Packmittel aus Polyethylen, Polypropylen oder Polyethylenterephthalat enthaltend eine erfindungsgemäße Zubereitung.

Das erfindungsgemäße Packmittel, in dem die erfindungsgemäße Zubereitung aufbewahrt wird, liegt erfindungsgemäß vorteilhaft in Form einer Tube oder einer Kunststoff-Flasche vor. Dabei sind Kunststoff-Flaschen erfindungsgemäß bevorzugt. Eine erfindungsgemäß besonders bevorzugte Ausführungsform stellen Kunststoff-Flaschen mit aufgesetztem Pumpspender dar, mit welchem die erfindungsgemäße Zubereitung aus dem Packmittel gefördert und ggf. versprüht werden kann.

Es ist erfindungsgemäß bevorzugt, wenn die erfindungsgemäße Zubereitung in einem Packmittel aus Polyethylen (PE) aufbewahrt wird. Erfindungsgemäß besonders bevorzugt ist dabei der Einsatz von "high density polyethylene"(HDPE nach DIN 7728, TI. 1, 01/1988).

Erfindungsgemäß ist insbesondere die Verwendung der erfindungsgemäßen Zubereitung zur Prophylaxe von Mückenstichen und Zeckenbissen.

Erfindungsgemäß ist die Verwendung von Verbindungen gewählt aus der Gruppe Hexylsalicylat, Linalylacetat, zur Abwehr von Mücken, Sandfliegen, Läusen, Bremsen, Wespen, Bienen, Fliegen und Zecken.

Erfindungsgemäß ist die Verwendung von Verbindungen gewählt aus der Gruppe Hexylsalicylat, Linalylacetat, zur Herstellung einer kosmetischen Zubereitung zur Abwehr von Mücken, Sandfliegen, Läusen, Bremsen, Wespen, Bienen, Fliegen und Zecken.

Die nachfolgenden Beispiele sollen die vorliegende Erfindung verdeutlichen ohne sie einzuschränken. Die Angaben beziehen sich stets auf Gewichts-%, sofern nicht andere Angaben gemacht werden.

| **W/O-Emulsionen** | | | | | |
|---|---|---|---|---|---|
| | **1** | **2** | **3** | **4** | **5** |
| Triglycerindiisostearat | 1,0 | 0,5 | 0,25 | 2,0 | 3,0 |
| Diglycerindipolyhydroxystearat | 1,0 | 1,5 | 1,75 | 3,0 | 2,0 |
| Paraffinöl | 12,5 | 10,0 | 8,0 | 5,0 | 7,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Decyloleat | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydrierte Rizinusöl | 0,5 | 0,75 | 1,0 | 2,5 | 5,0 |
| Cellulose | 0,5 | 1,0 | --- | 0,25 | 2,5 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 0,2 | 0.05 | 0.4 | 0.3 | 2,0 |
| Parfum | q,s | q,s | q,s | q,s | q,s |
| Ethanol | 2,0 | --- | 5,0 | --- | --- |
| Capryl-/Caprinsäuretriglycerid | 2,0 | 2,5 | 3,0 | 5,0 | 0,5 |
| Kaliumsorbat | 0,04 | 0,15 | 0,05 | 0,03 | 0,4 |
| Hexylsalicylat | 0,3 | 0,4 | 0,25 | 0,15 | 150ppm |
| Icaridin | 5 | 7,5 | 10 | 12,5 | 30 |
| Wasser | ad 100 | ad 100 | ad 100 | ad 100 | ad 100 |

| **W/O-Emulsionen** | | | | | |
|---|---|---|---|---|---|
| | **6** | **7** | **8** | **9** | **10** |
| PEG-30 Dipolyhydroxystearat | --- | 0,5 | 0,25 | --- | 8,0 |
| Lanolin Alcohol | 1,0 | 1,5 | 1,75 | 3,0 | -- |
| Paraffinöl | 12,5 | 10,0 | 8,0 | 5,0 | 12,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Hydriertes Polyisobuten | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Microcrystalline Cellulose | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Parfum | q,s | q,s | q,s | q,s | q,s |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Natriumdehydracet | --- | --- | 0,05 | --- | --- |
| Kaliumsorbat | 0,3 | 0,4 | 0,25 | 0,15 | --- |
| Linalylacetat | 0,15 | 15 ppm | 0,5 | 0,0025 | 0,0065 |
| Icaridin | 7,5 | 12,5 | 17,5 | 20 | 35 |
| Wasser | ad 100 | ad 100 | ad 100 | ad 100 | ad 100 |

| **W/S-Emulsionen** | | | | | |
|---|---|---|---|---|---|
| | **11** | **12** | **13** | **14** | **15** |
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| Cyclomethicon | 12,5 | 15 | 28,0 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Milchsäure | 0,2 | 0,1 | 0,2 | --- | --- |
| Natriumlactat | 0,2 | 1,0 | 0.05 | --- | --- |
| Parfum | q,s | q,s | q,s | q,s | q,s |
| Icaridin | 3,0 | 10 | 12,5 | 15 | 30 |
| Stearyldimethicon | 0,5 | --- | 0,7 | --- | --- |
| Dehydracetsäure | --- | --- | 0,05 | --- | 0,1 |
| Methylheptenone | 0,3 | 0,025 | 0,125 | 150 ppm | 0,75 |
| Wasser | ad 100 | ad 100 | ad 100 | ad 100 | ad 100 |

| **W/O-Emulsionen** | | | | | |
|---|---|---|---|---|---|
| | **16** | **17** | **18** | **19** | **20** |
| Polyglyceryl-2-dipolyhyroxystearat | 3,0 | --- | 0,25 | --- | 6,0 |
| Polyglyceryl-3 diisostearate | 1,0 | 3,5 | 1,75 | 2,5 | --- |
| PEG-40 sorbitanisostearate | --- | 2,5 | 0,5 | 3,5 | 3,0 |
| Paraffinöl | 12,5 | 10,0 | 8,0 | 5,0 | 17,5 |
| Isopropylstearat | 8,0 | 6,0 | 5,0 | 12,0 | 2,5 |
| hydrierte Kokosglyceride | 2,0 | 1,0 | 2,5 | 5,0 | 0,25 |
| Isopropylpalmitat | 0,5 | 1,0 | 0,75 | 3,0 | 0,25 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Diethylsuccinat | 1,618 | 16,18 | 7,5 | 15 | 40 |
| Icaridin | 1,618 | 16,18 | 7,5 | 15 | 40 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,1 | 0,3 | 1,0 |
| Natriumcitrat | 0,2 | 0,3 | 0,2 | 1,5 | 0.8 |
| Parfum | q,s | q,s | q,s | q,s | q,s |
| Capryl-/Caprinsäuretriglycerid | 2,0 | 2,5 | 3,0 | 5,0 | 0,5 |
| Kaliumsorbat | 0,24 | 0,15 | 0,05 | 0,3 | 0,4 |
| Wasser | ad 100 | ad 100 | ad 100 | ad 100 | ad 100 |

| **Silikon in Wasser-Emulsion** | | | | | |
|---|---|---|---|---|---|
| | **21** | **22** | **23** | **24** | **25** |
| Dimethiconcopolyol, Caprylic-Caprictriglycerid | 1,0 | 2,0 | 8,0 | 3,0 | 5,0 |
| Cyclomethicon | 12,5 | 15 | 25,0 | 10,0 | 7,5 |
| Dimethicon | 5,0 | 15,0 | 5,0 | 12,0 | 15,0 |
| Mineralöl | 0,5 | 0,75 | 1,0 | 2,0 | 0,25 |
| Phenyltrimethicon | 0,5 | 1,0 | 0,75 | 3,0 | 0,25 |
| Glycerin | 5,0 | 7,5 | 10,0 | 3,0 | 1,0 |
| Alpha-Isomethylionone | 0,1 | 40 ppm | 0,08 | 0,0045 | 0,15 |
| Icaridin | 10 | 20 | 30 | 40 | 15 |
| Xanthan Gum | --- | 0,1 | -- | 0,25 | 1,0 |
| Panthenol | 0,5 | 1,0 | 0,75 | 0,25 | 0,1 |
| Parfum | q,s | q,s | q,s | q,s | q,s |
| Methylparaben | 0,4 | 0,1 | 0,05 | 0,3 | 0,4 |
| Propylparaben | 0,3 | 0,4 | 0,25 | 0,15 | --- |
| Iodopropynylbutylcarbamat | --- | --- | 0,05 | --- | 0,1 |
| Wasser | ad 100 | ad 100 | ad 100 | ad 100 | ad 100 |

| **O/W-Emulsion** | | | | | |
|---|---|---|---|---|---|
| | **26** | **27** | **28** | **29** | **30** |
| Polyethylenglycol(21)stearylether | 1 | -- | 2,5 | 2 | 1,5 |
| Polyethylenglycol(2)stearylether | 1 | -- | 5,5 | 3 | 7,5 |
| Cetearylglucosid | --- | 8 | --- | --- | --- |
| Behenylalkohol | 3 | 2 | --- | 1 | --- |
| Stearylalkohol | 3 | 2 | --- | 2 | --- |
| Cetylstearylalkohol | 3 | 4 | --- | --- | 2 |
| hydriertes Polyisobuten | 0,5 | 0,75 | 1,0 | 2,0 | 0,25 |
| Icaridin | 10 | 20 | 15 | 7,5 | 5,0 |
| Octyldodecanol | 0,5 | 1,0 | 0,75 | 3,0 | 0,25 |
| Glycerin | 5 | 10 | 15 | 3 | 7,5 |
| Panthenol | 0,5 | 1,0 | 0,75 | 0,25 | 0,1 |
| Parfum | q,s | q,s | q,s | q,s | q,s |
| Methylparaben | 0,4 | 0,1 | 0,05 | 0,3 | 0,4 |
| Propylparaben | 0,3 | 0,4 | 0,25 | 0,15 | --- |
| Iodopropynylbutylcarbamat | --- | --- | 0,05 | --- | 0,1 |
| Hexylsalicylat | 0,1 | 20 ppm | 0,0065 | 0,0015 | 0,5 |
| Wasser | ad 100 | ad 100 | ad 100 | ad 100 | ad 100 |

| **O/W-Emulsionen** | | | | | |
|---|---|---|---|---|---|
| | **31** | **32** | **33** | **34** | **35** |
| Glycerylsteratcitrat | 1,0 | 0,5 | 1,5 | 9,0 | 0,3 |
| Polyethylenglycol(20)ceterarylether | 10,0 | 1,0 | 5 | -- | -- |
| Triglycerinmethylglucosedistearat | --- | --- | 3,5 | --- | 2,5 |
| Citronellyl Methylcrotonat | 0,75 | 1,5 | 2,25 | 0,125 | 0,035 |
| Icaridin | 7,5 | 15 | 22,5 | 30 | 37,5 |
| Dimethicon | 0,5 | 3,0 | 0,75 | 1,5 | 0,2 |
| Behenylalkohol | 1 | --- | 2 | --- | 0,2 |
| Dicaprylylcarbonat | 3 | 5 | 10 | 5 | 5 |
| Stearylalkohol | --- | --- | --- | 1 | 0,2 |
| Cetylstearylalkohol | --- | --- | 1 | 1 | 0,2 |
| Tocopherol | 0,5 | 0,5 | 0,75 | 0,25 | 0,1 |
| Octyldodecanol | 0,5 | --- | 0,75 | 3,0 | 0,25 |
| Panthenol | 0,5 | --- | 0,75 | 0,25 | 0,1 |
| Carbomer | 0,05 | 0,35 | 0,15 | 0,1 | --- |
| Parfum | q,s | q,s | q,s | q,s | q,s |
| Caprylic/Capric Triglycerid | 1 | 5 | 3 | 5 | 10 |
| Methylparaben | 0,4 | 0,3 | 0,05 | 0,3 | 0,4 |
| Propylparaben | 0,3 | --- | 0,25 | 0,15 | --- |
| Iodopropynylbutylcarbamat | --- | --- | 0,05 | --- | 0,1 |
| Phenoxyethanol | --- | 0,5 | --- | 0,15 | --- |
| Sorbitol | 10 | --- | -- | 5 | --- |
| Butylenglykol | --- | --- | --- | 5 | 10 |
| Propylenglykol | --- | --- | 10 | 5 | --- |
| Glycerin | --- | 7,5 | --- | --- | --- |
| Wasser | ad 100 | ad 100 | ad 100 | ad 100 | ad 100 |

| **Pumpspray** | | | | | |
|---|---|---|---|---|---|
| | **36** | **37** | **38** | **39** | **40** |
| Polyethylenglycol(40)stearat | 1,0 | --- | 0,5 | --- | --- |
| Cyclomethicon | 0,5 | --- | --- | --- | --- |
| Icaridin | 2 | 10 | 15 | 20 | 40 |
| Parfum | q,s, | q,s, | q,s, | q,s, | q,s, |
| Ethanol | 15 | 20 | 30 | 40 | 20 |
| Methylparaben | 0,4 | 0,1 | 0,05 | 0,3 | 0,4 |
| Meeresextrakt | --- | --- | 0,1 | --- | --- |
| Aloe Vera Extract | 0,1 | --- | 0,1 | --- | --- |
| Hammamelis-Extrakt | 0,1 | --- | 0,1 | 5 | 0,5 |
| Glycerin | 5 | 10 | 3 | 15 | 7,5 |
| Linalylacetat | 0,015 | 0,0065 | 35ppm | 0,15 | 0,0095 |
| Wasser | ad 100 | ad 100 | ad 100 | ad 100 | ad 100 |

| **Spay mit Repellent** | | | | | |
|---|---|---|---|---|---|
| | **41** | **42** | **43** | **44** | **45** |
| Ethylbutylacetylaminopropionat | 5 | 35 | 15 | 20 | 7,5 |
| N,N-Diethyltoluolamid | 5 | --- | --- | 1 | --- |
| Icaridin | 5 | 3,0 | 2,0 | 1,5 | 12,5 |
| 1-(3-Cyclohexen-1-yl-carbonyl)-2-methylpiperidin | 5 | --- | 2 | 1 | --- |
| Hammamelis-Extrakt | 0,2 | --- | --- | 1 | 0,2 |
| Aloe Vera Extract | --- | --- | 1 | 1 | 5,0 |
| Tocopherol | 0,5 | 0,5 | 0,75 | 0,25 | 0,1 |
| Meeresextrakt | 0,5 | --- | 0,75 | 3,0 | 0,25 |
| Panthenol | 0,5 | --- | 0,75 | 0,25 | 0,1 |
| Hexylsalicylat | 0,1 | 0,015 | 0,5 | 75 ppm | 0,0065 |
| Parfum | q,s, | q,s, | q,s, | q,s, | q,s, |
| Sorbitol | 10 | --- | --- | 5 | --- |
| Butylenglykol | --- | --- | --- | 5 | 10 |
| Propylenglykol | --- | --- | 10 | 5 | --- |
| Glycerin | --- | 7,5 | --- | --- | --- |
| Wasser | ad 100 | ad 100 | ad 100 | ad 100 | ad 100 |

## Patentansprüche

1. Kosmetische Zubereitung enthaltend
a) 1-Piperidincarboxylsäure 2-(2-hydroxyethyl)-1-methylpropylester (INN: Icaridin) sowie
b) ein oder mehrere Verbindungen gewählt aus der Gruppe Hexylsalicylat, Linalylacetat, Methylheptenon, alpha-Isomethylionon, Butylphenylmethylpropional, Coumarin, Hexylcinnamal, Diethylsuccinat, Hydroxyisohexyl 3-Cyclohexencarboxaldehyd und Citronellylmethylcrotonat.

2. Kosmetische Zubereitung enthaltend
a) 0,1 bis 40 Gewichts-% 1-Piperidincarboxylsäure 2-(2-hydroxyethyl)-1-methylpropylester sowie
b) 1 ppm bis 2,5 Gewichts-% Hexylsalicylat,
jeweils bezogen auf das Gesamtgewicht der Zubereitung.

3. Kosmetische Zubereitung enthaltend
a) 0,1 bis 40 Gewichts-% 1-Piperidincarboxylsäure 2-(2-hydroxyethyl)-1-methylpropylester sowie
b) 1ppm bis 2,5 Gewichts-% Linalylacetat,
jeweils bezogen auf das Gesamtgewicht der Zubereitung.

4. Kosmetische Zubereitung enthaltend
a) 0,1 bis 40 Gewichts-% 1-Piperidincarboxylsäure 2-(2-hydroxyethyl)-1-methylpropylester sowie
b) 1 ppm bis 2,5 Gewichts-% 2-Isobutyl-4-hydroxy-4-methyltetrahydropyran,
jeweils bezogen auf das Gesamtgewicht der Zubereitung.

5. Kosmetische Zubereitung nach einem der vorhergehenden Ansprüche, **dadurch gekennzeichnet, dass** die kosmetische Zubereitung in Form einer Emulsion vorliegt.

6. Packmittel aus Polyethylen, Polypropylen oder Polyethylenterephthalat enthaltend eine Zubereitung nach einem der vorhergehenden Ansprüche.

7. Tuch oder Pflaster getränkt mit einer Zubereitung nach einem der vorhergehenden Ansprüche.

## Claims

1. Cosmetic preparation comprising
a) 2-(2-hydroxyethyl)-1-methylpropyl 1-piperidinecarboxylate (INN: icaridin) and
b) one or more compounds selected from the group consisting of hexyl salicylate, linalyl acetate, methylheptenone, alpha-isomethylionone, butylphenylmethylpropional, coumarin, hexylcinnamal, diethyl succinate, hydroxyisohexyl 3-cyclohexenecarboxaldehyde and citronellyl methylcrotonate.

2. Cosmetic preparation comprising
a) 0.1 to 40% by weight of 2-(2-hydroxyethyl)-1-methylpropyl 1-piperidinecarboxylate and
b) 1 ppm to 2.5% by weight of hexyl salicylate,
in each case based on the total weight of the preparation.

3. Cosmetic preparation comprising
a) 0.1 to 40% by weight of 2-(2-hydroxyethyl)-1-methylpropyl 1-piperidinecarboxylate and
b) 1 ppm to 2.5% by weight of linalyl acetate,
in each case based on the total weight of the preparation.

4. Cosmetic preparation comprising
a) 0.1 to 40% by weight of 2-(2-hydroxyethyl)-1-methylpropyl 1-piperidinecarboxylate and
b) 1 ppm to 2.5% by weight of 2-isobutyl-4-hydroxy-4-methyltetrahydropyran, in each case based on the total weight of the preparation.

5. Cosmetic preparation according to one of the preceding claims, **characterized in that** the cosmetic preparation is in the form of an emulsion.

6. Packaging made of polyethylene, polypropylene or polyethylene terephthalate comprising a preparation according to one of the preceding claims.

7. Wipe or plaster impregnated with a preparation according to one of the preceding claims.

## Revendications

1. Préparation cosmétique contenant
a) l'ester 2-(2-hydroxyéthyl)-1-méthylpropylique de l'acide 1-pipéridinecarboxylique (INN : Icaridine) ainsi que
b) un ou plusieurs composés choisis dans le groupe formé par le salicylate d'hexyle, l'acétate de linalyle, la méthylhepténone, l'alpha-isométhylionone, le butylphénylméthylpropiolal, la coumarine, l'hexylcinnamal, le succinate de diéthyle, l'hydroxyisohexyl-3-cyclohexènecarboxaldéhyde et le crotonate de citronellylméthyle.

2. Préparation cosmétique contenant
a) 0,1 à 40% en poids d'ester 2-(2-hydroxyéthyl)-1-méthylpropylique de l'acide 1-pipéridinecarboxylique ainsi que
b) 1 ppm à 2,5% en poids de salicylate d'hexyle,
à chaque fois par rapport au poids total de la composition.

3. Préparation cosmétique contenant
a) 0,1 à 40% en poids d'ester 2-(2-hydroxyéthyl)-1-méthylpropylique de l'acide 1-pipéridinecarboxylique ainsi que
b) 1 ppm à 2,5% en poids d'acétate de linalyle,
à chaque fois par rapport au poids total de la composition.

4. Préparation cosmétique contenant
a) 0,1 à 40% en poids d'ester 2-(2-hydroxyéthyl)-1-méthylpropylique de l'acide 1-pipéridinecarboxylique ainsi que
b) 1 ppm à 2,5% en poids de 2-isobutyl-4-hydroxy-4-méthyltétrahydropyranne, 1
à chaque fois par rapport au poids total de la composition.

5. Préparation cosmétique selon l'une quelconque des revendications précédentes, **caractérisée en ce que** la préparation cosmétique se trouve sous forme d'une émulsion.

6. Emballage en polyéthylène, en polypropylène ou en poly(téréphtalate d'éthylène) contenant une préparation selon l'une quelconque des revendications précédentes.

7. Tissu ou emplâtre imbibé d'une préparation selon l'une quelconque des revendications précédentes.
